# EUROPEAN PATENT SPECIFICATION

(11) **EP 1 509 123 B1**
(45) Date of publication and mention of the grant of the patent: **22.07.2009**
(21) Application number: 03727968.4
(22) Date of filing: 21.05.2003
(51) Int. Cl.: A61B 3/16

(54) **APPARATUS AND METHOD OF MEASURE OF THE CORRECTED INTRAOCULAR PRESSURE**
VORRICHTUNG UND VERFAHREN ZUR MESSUNG DES KORRIGIERTEN AUGENINNENDRUCKS
APPAREIL ET PROCEDE DE MESURE DE LA PRESSION INTRA-OCULAIRE CORRIGEE

(30) Priority: 22.05.2002 IT CS20020003; 21.05.2003 IT CZ20030011
(43) Date of publication of application: 02.03.2005
(73) Proprietor: Lucente, Amedeo Francesco Antonio, 87012 Castrovillari (IT)
(72) Inventor: Lucente, Amedeo Francesco Antonio, 87012 Castrovillari (IT)
(86) International application number: PCT/IT2003/000306
(87) International publication number: WO 2003/096888

(56) References cited:
- EP-A- 1 147 738
- US-A- 5 474 066
- US-A- 6 113 542

## Description

### Technical field of the invention

Object of the present invention is an apparatus and a method for the evaluation of the intraocular pressure corrected with the measure of the corneal thickness and curvature, of the anterior chamber volume and depth of the eye ball under examination.

### State of the art

The intraocular pressure is largely considered as the most important parameter in the diagnosis of ocular hypertension and glaucoma. H. Goldmann's applanation tonometer is considered as a standard apparatus to determine this important and essential parameter. Applanation tonometry is based on Imbert-Fick physical principle subsequently modified by Goldmann, according to him, the cornea, for the flattened surface of 7.35 mm², is considered a surface which has membrane characteristics, that is to say without flexional stiffness. Goldmann considered in fact that the piston power flattens the cornea as if there was no resistance, since the two contrasting forces of the corneal stiffness and of the capillarity, that is of the surface tension, are equal; this is due to the small dimension of the corneal surface concerned with the applanation piston.

Studying systems with flexional stiffness, it has been found that flexional stiffness changes according to the cube of the structure thickness under observation and it changes according to the curvature of the structure as well as to the different thickness we find in the periphery rather than in the apex. These mathematical relations show that if we do not consider either the cornea morphology or the anatomy of the anterior segment, we can easily get tonometric measures but far from reality. Under these circumstances, one could start an unnecessary anti-hypertension therapy with pressures of 22-26 mmHg, in corneas thicker than 600 micrometers or, vice versa, wrongly considering good pressures of 16-18 mmHg in corneas having a thickness of 450 micrometers or less; with high risks for the papillary system of these patients. Such mistakes are enhanced by the actual non-contact tonometers, in comparison with the applanation tonometer of Goldmann, particularly with very high values of the thickness and/of the curvature of the cornea.

In order to avoid these inconveniences, apparatuses that take in account also other parameters for the evaluation of the intraocular pressure have been studied. Patent US-B-5,131,739 describes an apparatus to measure the intraocular pressure and the curvature. A non-contact tonometer has been used. This apparatus is used to make two separate measurements and keratometric data are not used to correct those values found by the non-contact tonometer.

The Patent US 5,474,066 describes a non-contact tonometer comprising one videocamera for aligning the eye, means for measuring the thickness of the cornea and means for measuring the depth of the anterior chamber. The intraocular pressure is calculated by also taking into account the detected corneal thickness.

In patent US-B-6,113,542, the inventor describes an apparatus comprising an applanation tonometer and an ophthalmic pachymeter which has a pachymetric probe and a microprocessor. While in use, the applanation tonometer produces an applanation signal indicating intraocular pressure. The ophthalmic pachymeter produces a pachymetric signal indicating corneal central thickness. The microprocessor is programmed to modify the eye pressure value according to the corneal central thickness. The described apparatus, uses the applanation tonometer which has the disadvantage to compel the patient to keep his eye wide open and still for several seconds. Furthermore, while correcting the intraocular pressure value, this apparatus does not consider the curvature, the values of the depth and the volume of the anterior chamber of the eye ball.

Besides it does not respect any parameter of sterility, since it is in contact with the cornea just like all applanation tonometers. The present invention has the aim to overcome difficulties and disadvantages present in the solutions which are now on the market.

Main purpose of the present invention is to create an apparatus of measure of the corrected intraocular pressure according to the individual morphologic and anatomic parameters of the eye ball under examination, characterized by the fact that it is constituted by a non-contact tonometer, by two videocameras set on both sides of the central probe in oblique position as to the central nozzle of the non-contact tonometer, but on the same horizontal plane passing through said central nozzle, by a laminar light (fissure), by a microprocessor and by a database containing all data about measures of intraocular pressure, thickness and curvature of the cornea, volume and depth of the eyeball anterior chamber and that, through a suitable algorithm, it corrects the value of the measured intraocular pressure in function of the intraocular pressure initially detected and of the anatomic measures above mentioned, and it is updated using the detected data and the values of the so corrected intraocular pressure.

Another major characteristic is to realize a method of measure of the corrected intraocular pressure where the apparatus according to the invention is drawn near the cornea and it is controlled by a lever of the patient modulus, so that at the proper distance the perfectly focused cornea under examination is shown on the monitor, where the evaluation of the correct distance at which measures are to be done, is detected by the two videocameras set on both sides of the non-contact tonometer in oblique position as to the direction of the tonometer whiff, but on the same horizontal plane containing the two videocameras and the direction of the tonometer whiff, and a first pilot-whiff is directed onto the cornea of the eye ball under examination; the microprocessor will automatically and respectively activate the phases of measuring thickness of cornea, curvature of cornea, volume and depth of the examined eye ball anterior chamber; these measures are recorded and processed by the microprocessor which sends them to the database and this microprocessor, once it has received the measure of the intraocular pressure detected with the pilot-whiff, sends to the tonometer a second command in order to measure the intraocular pressure with whiffs of an intensity calibrated in function of the recorded bulbar deformation; these whiffs will be sent if the operator keeps its focalization on the cornea, that is displayed on the monitor; the values of the intraocular pressure resulting with the new calibrated whiffs, are sent to the microprocessor which first records them in the database, separately and as average, and then it calculates the values of the intraocular pressure with the calibrated whiffs; subsequently the microprocessor sends to the display the values of measures of the: thickness of cornea, curvature of cornea, volume and depth of the examined eye ball anterior chamber, intraocular pressure with pilot-whiff as previously described, intraocular pressure with calibrated whiff according to the corneal deformation recorded and, if that is the case further corrected in relation to the values of thickness and curvature of cornea, volume and depth of the eye ball anterior chamber under examination.

Another characteristic is given by the fact that the whiff can be modulated not only in function of the value of the intraocular pressure initially recorded, but also in function of the anatomical parameters of the eye ball previously detected; both the intraocular pressure with the modulated whiff and the intraocular pressure with the modulated whiff corrected in function of the anatomical bulbar parameters detected, will be shown on the display.

Another characteristic is given by the fact that whiffs modulated in function of the value of the initial intraocular pressure and of anatomical parameter of the eye ball previously measured, are three whiffs of which one is of higher intensity and an other is of lower intensity than the intensity of the modulated corrected whiff, in order to determine individually the elastic module of the patient cornea, that will be put in evidence on the display.

Further characteristics and advantages of the present invention will be clearer thanks to the following description of a way to realize the invention given as non limitative example from figures 1, 2 and 3.

### Brief description of the figures

Figure 1 represents the apparatus, object of the present invention, in a schematic form;
Figure 2 shows the block diagram of the method, object of the present invention;
Figure 3 shows another block diagram of the method, object of the present invention.

### Detailed description of the invention

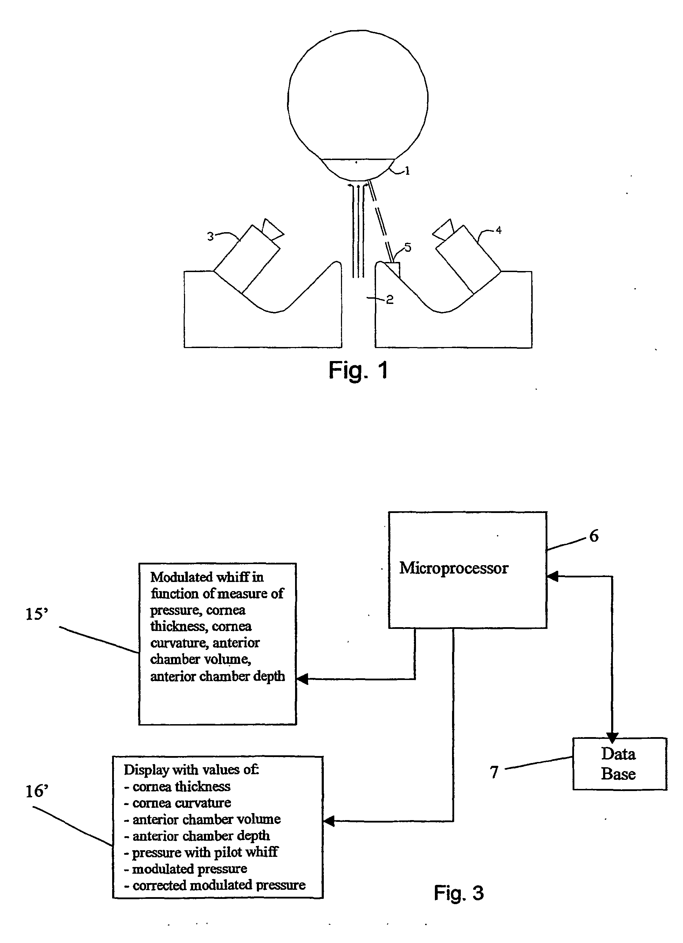

Referring to figure 1, the apparatus and method of measure of the cornea corrected intraocular pressure (1), object of the present invention, is constituted by a non-contact tonometer (2), two videocameras (3) and (4), a laminar light (fissure) (5), mobile if necessary, a microprocessor (6) and a database (7) containing all data of intraocular pressure measures, thickness and curvature of the cornea, volume and depth of the eyeball anterior chamber and that, through a suitable algorithm, it corrects the value of the intraocular pressure measured in function of the anatomic measures above mentioned, and it is updated using the detected data and the values of the corrected intraocular pressure.

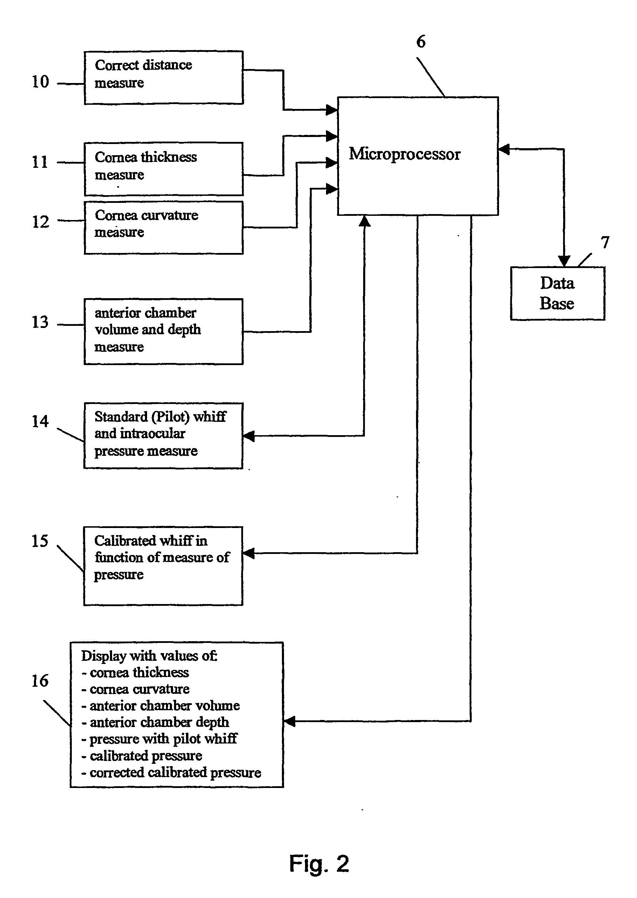

Referring to figure 2, the block diagram of the working way of the apparatus and method of measure of the corrected intraocular pressure, object of the present invention.

In phase (10) the apparatus is drawn near the eyeball cornea (1) and, by a lever, the displacement of the patient modulus is controlled. At the proper distance we can see on the monitor that the cornea under examination is perfectly focused. The evaluation of the correct distance at which measures are to be done, is detected by the two video-cameras (3) and (4) set on both sides of the non-contact tonometer (2), in oblique position as to the direction of the tonometer whiff, but on the same horizontal plane containing the two videocameras (3) and (4) and the direction of the tonometer whiff. At the correct distance a first command is sent to the microprocessor (6) which activates automatically and in succession the phases of pilot-whiffing and measuring the initial intraocular pressure(11), just like the present non-contact tonometers on the market, the phases of measuring thickness of cornea (12), curvature of cornea (13), volume and depth of the eyeball anterior chamber (14) under examination. All these measures are sent to the microprocessor (6) which in its turn sends them to the database (7). Said microprocessor (6), once it has received the measure of the initial intraocular pressure, sends to the tonometer (2) a second command (15) in order to measure the intraocular pressure with whiffs of an intensity calibrated in function of the bulbar deformation initially detected; these whiffs will be sent if the operator keeps the cornea focalisation, that is displayed on the monitor. These whiffs will be calibrated so to avoid an excessive massage and stress of the eyeball and its content, which being incompressible, will partly flow through the natural via of trabecular defluxion. In this way the apparatus will not uselessly stimulate the eyeball more than the pressure initially verified, so to avoid the excessive excitement of the aqueous humor in the anterior chamber and so recording wrong values of the intraocular pressure. Values of the intraocular pressure with calibrated whiffs are sent to the microprocessor (6) which first records them in the database (7) separately and as average, and then it calculates the value of the intraocular pressure with a whiff calibrated of the eyeball under examination and of the intraocular pressure with a whiff calibrated and corrected by the anatomical individual parameters of the single patient. Subsequently the microprocessor (6) sends to the display (16) the values of measures of the thickness of cornea, curvature of cornea, volume and depth of the eyeball anterior chamber, intraocular pressure with pilot-whiff, with calibrated whiff as previously described and with calibrated and corrected whiff using the values of thickness and curvature of cornea and of the volume and depth of the eyeball anterior chamber. The datum of the intraocular pressure with calibrated whiff will be more reliable than the one presently obtained with modem tonometers on the market, since it modulates the whiff according to the needs of the intraocular pressure to detect without massaging excessively the eyeball and its content, which being incompressible, will partly flow through the natural via of trabecular defluxion.

Referring to figure 3, the tonometer will also be able to modulate the whiff in function of the value of the measure of the initial intraocular pressure and of the anatomical parameters of the eyeball previously detected (15').

The intraocular pressure with modulated whiff and the intraocular pressure with modulated whiff corrected (16') through the aforesaid algorithm in function of the anatomical measures previously mentioned, in addition to the anatomical parameters of the eyeball previously detected, will be shown on the display.

A further embodiment of the measuring method of the corneal corrected intraocular pressure(1) is to modulate, in function of the values of the initial intraocular pressure and of eyeball parameters previously measured, three whiffs one of which has an intensity higher and an other one has a lower intensity than the intensity of the modulated corrected whiff, in order to determine the elastic modulus (Young's modulus) of each single patient which will be put in evidence on the display.

## Claims

1. Apparatus of measure of the eyeball corrected intraocular pressure (1) comprising a non-contact tonometer (2), two videocameras (3 and 4) set on both sides of the central probe in oblique position as to the central nozzle of the non-contact tonometer (2), but on the same horizontal plane passing through the central nozzle, a laminar light on the corneal arch (5) under examination, a microprocessor (6) a database (7) containing all data about anatomical measures of intraocular pressure, thickness and curvature of the cornea, volume and depth of the eyeball anterior chamber, means for correcting, through a suitable algorithm, the value of the measured intraocular pressure in function of the intraocular pressure initially detected and of the anatomical measures previously mentioned and wherein the apparatus is updated using the detected data and the values of the so corrected intraocular pressure.

2. Apparatus of measure of the eyeball corrected intraocular pressure (1) according to claim 1, **characterized by** the fact that the laminar light is able to rotate of an angle of 180°.

3. Method of measure of the eyeball corrected intraocular pressure(1) in which the apparatus, according to claim 1 or 2, is drawn near the cornea of the eyeball (1) and, by a lever, the displacement of a patient modulus is controlled, in which at the proper distance the perfectly focused cornea under examination appears on the monitor; where the evaluation of the correct distance to which measures are to be done, is detected by the videocameras (3) and (4) set on both sides of the non-contact tonometer (2), in oblique position as to the direction of the whiff of tonometer (2), but on the same horizontal plane containing the two video-cameras (3) and (4) and the direction of the tonometer whiff; wherein at the correct distance a first command is sent to the microprocessor (6) which will automatically and respectively activate the phases of pilot-whiffing and the phases of measuring the intraocular pressure (11), thickness of cornea (12), curvature of cornea (13), volume and depth of the eyeball anterior chamber (14) under examination; wherein all these measures are sent to the microprocessor (6) which in its turn sends them to the database (7); wherein said microprocessor (6), once it has received the measure of the intraocular pressure with pilot-whiffing, sends to the tonometer (2) a second command (15) in order to measure the intraocular pressure with whiffs of an intensity calibrated according to the bulbar deformation recorded; wherein these whiffs will be sent if the operator keeps its focalization on the cornea, displayed on the monitor; wherein the values of the intraocular pressure with calibrated whiffs are sent to the microprocessor (6) which first records them in the database (7) separately and as average, and then it calculates the value of the intraocular pressure with whiff calibrated in function of the initial intraocular pressure corrected and of the detected anatomical data; wherein subsequently the microprocessor (6) sends to the display (16) the values of measures of the: thickness of cornea, curvature of cornea, volume and depth of the eyeball anterior chamber, intraocular pressure with pilot-whiff, intraocular pressure with calibrated whiff and intraocular pressure with calibrated whiff corrected through an algorithm using the values of thickness and curvature of cornea, volume and depth of the examined eyeball anterior chamber.

4. Method of measure of the eyeball corrected intraocular pressure (1) according to claim 3, **characterized by** the fact that the whiff is modulated not only in function of the value of the measure of the initial intraocular pressure, but also of the eyeball anatomical parameters previously detected (15') and that the intraocular pressure with modulated whiff and the intraocular pressure with modulated whiff corrected (16') through an algorithm in function of the anatomical measures above mentioned, in addition to the anatomical parameters of the eyeball previously detected, will be shown on the display.

5. Method of measure of the eyeball corrected intraocular pressure (1) according to claim 4, **characterized by** the fact that said modulated whiffs, in function of the values of the initial intraocular pressure and of eyeball anatomical parameters previously measured, are three modulated whiffs, one of which is of higher intensity and an other is of lower intensity than the intensity of the modulated corrected whiff, in order to determine the elastic modulus of the patient which will be put in evidence on the display.

## Patentansprüche

1. Vorrichtung zur Messung des korrigierten Augeninnendrucks, umfassend ein kontaktloses Tonometer (2), zwei Videokameras (3 und 4), die zu beiden Seiten des zentralen Messkopfs schräg zur zentralen Düse des kontaktlosen Tonometers (2) in einer gemeinsamen und durch die zentrale Düse verlaufenden Horizontalebene angeordnet sind, ein auf den zu untersuchenden Corneabogen gerichtetes, laminares Licht, einen Mikroprozessor (6), eine Datenbank (7), die alle Daten bezüglich anatomischer Maße und Augeninnendruck. Dicke und Krümmung der Hornhaut sowie Volumen und Tiefe der vorderen Augenkammer enthält, und Mittel zur Korrektur des Wertes des gemessenen Augeninnendrucks mittels eines geeigneten Algorithmus in Funktion des anfänglich ermittelten Augeninnendrucks und der zuvor erwähnten anatomischen Maße, wobei die Vorrichtung durch die Verwendung der erfassten Daten und die Werte des auf diese Weise korrigierten Augeninnendrucks upgedated wird.

2. Vorrichtung zur Messung des korrigierten Augeninnendrucks nach Anspruch 1, **dadurch gekennzeichnet, dass** das laminare Licht um einen Winkel von 180° schwenkbar ist.

3. Verfahren zur Messung des korrigierten Augeninnendrucks, bei dem die Vorrichtung nach Anspruch 1 oder 2 in die Nähe der Cornea des Augapfels gebracht wird und mittels eines Hebels die Verschiebung eines Patientenmodulus gesteuert wird, worauf nach Erreichen des korrekten Abstandes die zu prüfende, exakt fokussierte Cornea auf dem Monitor erscheint, wobei die Auswertung des korrekten Abstandes, bei dem die Messungen durchzuführen sind, durch die Videokameras (3, 4) erfasst wird, welche zu beiden Seiten des kontaktlosen Tonometers (2) schräg zur Blasrichtung des Tonometers (2) und in einer gemeinsamen, die beiden Videokameras (3, 4) und die Tonometerblasrichtung enthaltenden Horizontalebene angeordnet sind, wobei bei korrektem Abstand ein erster Befehl an den Mikroprozessor (6) abgegeben wird, der automatisch die jeweiligen Phasen eines Pilotblasstrahls und die Messphasen zur Bestimmung des Augeninnendrucks (11), der Hornhautdicke (12), der Hornhautkrümmung (13) und des Volumens und der Tiefe der zu prüfenden, vorderen Augenkammer (14) aktiviert, wobei alle diese Messwerte an den Mikroprozessor (6) weitergegeben werden, der diese seinerseits zur Datenbank (7) sendet, wobei der Mikroprozessor (6) nach Erhalt des über den Pilotblasstrahl ermittelten Messwertes for den Augeninnendruck an das Tonometer (2) einen zweiten Befehl (15) abgibt, um den Augeninnendruck mit Blasstrahlen einer entsprechend der gespeicherten Bulbusverformung kalibierten Intensität zu messen, wobei diese Blasstrahlen abgegeben werden, wenn die Bedienungsperson deren auf dem Monitor angezeigte Fokussierung auf die Cornea beibehält, wobei die Werte des Augeninnendrucks aufgrund der kalibrierten Blasstrahlen an den Mikroprozessor (6) gesandt werden, der sie zunächst separat und als Mittelwert in die Datenbank (7) einspeichert und dann den Wert des Augeninnendruckes über den kalibrierten Blasstrahl in Funktion des korrigierten anfänglichen Augeninnendrucks und der erfassten anatomischen Daten errechnet, worauf der Mikroprozessor (6) an das Display (16) die Messwerte für die Dicke und Krümmung der Hornhaut, das Volumen und die Tiefe der vorderen Augenkammer, den mit Pilotblasstrahl ermittelten Augeninnendruck, den mit kalibriertem Blasstrahl ermittelten Augeninnendruck und den mit kalibriertem Blasstrahl über einen Algorithmus unter Verwendung der Werte für die Dicke und Krümmung der Hornhaut sowie für das Volumen und die Tiefe der untersuchten vorderen Augenkammer korrigierten Augeninnendruck weiterleitet.

4. Verfahren zur Messung des korrigierten Augeninnendrucks nach Anspruch 3, **dadurch gekennzeichnet, dass** der Blasstrahl nicht nur in Funktion von der Messung des anfänglichen Augeninnendrucks moduliert wird, sondern auch von den zuvor erfassten anatomischen Parametern (15') des Augapfels, und dass der mit moduliertem Blasstrahl ermittelte Augeninnendruck und der mit moduliertem Blasstrahl und mittels eines Algorithmus in Funktion von den angegebenen anatomischen Messwerten korrigierte Augeninnendruck (16') zusätzlich zu den zuvor ermittelten anatomischen Parametern des Augapfels auf dem Display angezeigt werden.

5. Verfahren zur Messung des korrigierten Augeninnendrucks nach Anspruch 4, **dadurch gekennzeichnet, dass** die modulierten Blasstrahlen in Funktion von den Werten für den anfänglichen Augeninnendruck und von den zuvor ermittelten anatomischen Parametern des Augapfels aus drei modulierten Blasstrahlen bestehen, von denen einer eine höhere Intensität und ein anderer eine geringere Intensität als die Intensität des korrigierten modulierten Blasstrahls hat, um den elastischen Modul des Patienten zu bestimmen und diesen auf dem Display anzuzeigen.

## Revendications

1. Appareil de mesure de la pression intraoculaire corrigée du globe oculaire (1) comprenant un tonomètre sans contact (2), deux caméras vidéo (3) et (4) placées des deux côtés de la sonde centrale en position oblique par rapport à la buse centrale du tonomètre sans contact (2) mais sur le même plan horizontal passant par la buse centrale, une lumière laminaire sur l'arc cornéen (5) examiné, un microprocesseur (6) et une base de données (7) contenant toutes les données sur les mesures anatomiques de la pression intraoculaire, l'épaisseur et la courbure de la cornée, le volume et la profondeur de la chambre antérieure du globe oculaire, des moyens de correction par un algorithme approprié, de la valeur de la pression intraoculaire mesurée en fonction de la pression intraoculaire détectée initialement et des mesures anatomiques mentionnées auparavant, et dans lequel l'appareil est actualisé en utilisant les données détectées et les valeurs de la pression intraoculaire ainsi corrigée.

2. Appareil de mesure de la pression intraoculaire corrigée du globe oculaire (1) selon la revendication 1, **caractérisé en ce que** la lumière laminaire peut tourner sur un angle de 180°.

3. Procédé de mesure de la pression intraoculaire corrigée du globe oculaire (1) dans lequel l'appareil selon la revendication 1 ou 2 est approché de la cornée du globe oculaire (1) et par un levier, le déplacement d'un module du patient est contrôlé, dans lequel, à distance appropriée, la cornée parfaitement focalisée qui est examinée apparaît sur le moniteur où l'évaluation de la distance correcte à laquelle les mesures doivent être effectuées est détectée par les caméras vidéo (3) et (4) fixées des deux côtés du tonomètre sans contact (2) en position oblique par rapport à la direction du jet d'air du tonomètre (2) mais sur le même plan horizontal contenant les deux caméras vidéo (3) et (4) et dans le sens du jet d'air du tonomètre, dans lequel à distance la correcte, un premier ordre est envoyé au microprocesseur (6) qui activera automatiquement et respectivement les phases de projection d'air pilote et les phases de mesure de la pression intraoculaire (11), de l'épaisseur de la cornée (12), de la courbure de la cornée (13), du volume et de la profondeur de la chambre antérieure du globe oculaire (14) qui est examiné ; toutes ces mesures étant envoyées au microprocesseur (6) qui les envoie lui-même à la base de données (7) ; dans lequel ledit microprocesseur (6), lorsqu'il a reçu la mesure de la pression intraoculaire avec la projection d'air pilote, envoie au tonomètre (2) un deuxième ordre (15) afin de mesurer la pression intraoculaire avec des jets d'air d'une intensité étalonnée selon la déformation bulbaire enregistrée ; ces jets d'air étant envoyés si l'opérateur maintient la focalisation sur la cornée, affichée sur le moniteur; les valeurs de la pression intraoculaire avec les jets d'air étalonnés étant envoyées au microprocesseur (6) qui les enregistré tout d'abord dans la base de données (7), séparément et sous forme de moyenne, puis calcule la valeur de la pression intraoculaire avec le jet d'air étalonné de la pression intraoculaire initiale corrigée et des données anatomiques détectées ; ensuite, le microprocesseur (8) envoie à l'écran (16) les valeurs de mesures de l'épaisseur de la cornée, la courbure de la cornée, le volume et la profondeur de la chambre antérieure du globe oculaire, la pression intraoculaire avec jet d'air pilote, la pression intraoculaire avec jet d'air étalonné et la pression intraoculaire avec jet d'air étalonné corrigée par un algorithme en utilisant les valeurs d'épaisseur et de courbure de la cornée, le volume et la profondeur de la chambre antérieure du globe oculaire examiné.

4. Procédé de mesure de la pression intraoculaire corrigée du globe oculaire (1) selon la revendication 3, **caractérisé en ce que** le jet d'air est modulé non seulement en fonction de la valeur de mesure de la pression intraoculaire initiale mais également des paramètres anatomiques du globe oculaire détectés auparavant (15') et **en ce que** la pression intraoculaire avec jet d'air modulé et la pression intraoculaire avec jet d'air corrigé modulé corrigée (16') par un algorithme en fonction des mesures anatomiques mentionnées ci-dessus, en plus des paramètres anatomiques du globe oculaire détectés au préalable, seront affichées sur l'écran.

5. Procédé de mesure de la pression intraoculaire corrigée du globe oculaire (1) selon la revendication 4, **caractérisé en ce que** lesdits jets d'air modulés en fonction des valeurs de la pression intraoculaire initiale et des paramètres anatomiques du globe oculaire mesurés auparavant, sont trois jets d'air modulés, l'un étant d'intensité supérieure et l'autre étant d'intensité inférieure à l'intensité du jet d'air modulé corrigée afin de déterminer le module d'élasticité du patient qui sera mis en évidence sur l'écran.
